# EUROPÄISCHE PATENTSCHRIFT

(11) **EP 1 448 259 B1**
(45) Veröffentlichungstag und Bekanntmachung des Hinweises auf die Patenterteilung: **19.09.2007**
(21) Anmeldenummer: 02791710.3
(22) Anmeldetag: 25.11.2002
(51) Int. Cl.: A61M 25/00

(54) **BLASENKATHETER**
BLADDER CATHETER
SONDE VESICALE

(30) Priorität: 27.11.2001 DE 10158091; 25.11.2002 DE 10255065
(43) Veröffentlichungstag der Anmeldung: 25.08.2004
(73) Patentinhaber: Microcuff GmbH, 69469 Weinheim (DE)
(72) Erfinder: GÖBEL, Fred, 69259 Wilhelmsfeld (DE)
(74) Vertreter: Küchler, Stefan
(86) Internationale Anmeldenummer: PCT/EP2002/013211
(87) Internationale Veröffentlichungsnummer: WO 2003/045487

(56) Entgegenhaltungen:
- EP-A- 0 935 977
- WO-A-00/35358
- WO-A-97/25093
- US-A- 5 417 657

## Beschreibung

### Technisches Gebiet

Die Erfindung richtet sich auf einen Blasenkatheter zur transurethralen oder suprabubischen Einführung in die Hamblase, bestehend aus einem elastischen Katheterschaft mit einem daran befestigten, auffüllbaren Ballonelement aus Polyurethan oder einer Polyurethan-Polyvinylchlorid-Mischung oder vergleichbarem material auf Polyurethanbasis, das über wenigstens eine Füllöffnung mit einem in die Wand des Katheterschaftes eingebundenen Auffüllkanal kommuniziert und durch Schaftstücke mit dem Katheterschaft verbunden ist.

### Stand der Technik

In der medizinischen Versorgung werden Blasenkatheter häufig angewendet. Die im Gebrauch befindlichen Blasenkatheter bestehen aus einem elastischen Katheterschaft, an dessen vorderem, in der Harnblase platzierten Ende ein mit Flüssigkeit aufffüllbares Ballonelement angebracht ist. Der Katheterschaft verfügt über einen Auffüllkanal, der über eine Öffnung in der Katheterwand in das Balloninnere mündet. Das Ballonelement dient vorrangig zur sicheren mechanischen Verankerung des Katheters in der Harnblase. Im Blasenausgang platziert hat der Ballon zudem eine gewisse dichtende Funktion und verhindert, dass Urin aus der Harnblase am Katheter vorbei durch die Harnröhre abläuft.

Im nicht gefüllten Zustand entspricht das Ballonelement einer über den Katheterschaft gezogenen Manschette, welche dem Schaft allseitig, in der Regel unter leichter Spannung, in jedem Falle jedoch faltungsfrei anliegt. Die Manschette entspricht einem Schlauchstück, welches in der Regel aus dem gleichen Material oder einem gleichartigen, in seinem Dehnungsverhalten modifizierten Material wie der Schaft gefertigt ist. Konventionelle Ballonelemente werden in dieser bestimmten, dem Schaft im entleerten Zustand eng anliegenden Bauart ausgeführt, um das Ballonelement mit möglichst geringem Widerstand durch die Harnröhre in das Blasenlumen vorschieben zu können. Schmerzhafte Irritationen oder Läsionen der Harnröhren-Schleimhaut beim Einführen des Katheters durch zuvor bestehende bzw. sich beim Vorschieben ausformende Falten oder Wülste in der Wandung des Ballonelements werden so vermieden. Nachdem das Ballonelement sicher in die Blase eingeführt wurde, wird die dem Schaft eng aufsitzende Manschette (Ballon element) mit einer Flüssigkeit unter relativ hohem Druck zum Ballon aufgedehnt. Das bei konventionellen Kathetern für den Katheterschaft und das Ballonelement ausgewählte Material, in der Regel Latex oder Silikon, lässt eine Aufdehnung des Ballonelements auf ein Volumen von 5 bzw. 30 ml zu. In der klinischen Praxis werden üblicherweise Blasenkatheter mit diesen beiden Ballonvolumina verwendet.

Idealerweise retrahiert sich das zum Ballon aufgedehnte Ballonelement auch nach längerfristigem Gebrauch des Katheters völlig und liegt dem Katheterschaft als manschettenartiges Schlauchstück ohne Ausbildung von Falten oder Wülsten eng an, so dass das entleerte Ballonelement auch beim Zurückziehen des Katheters durch die Harnröhre am empfindlichen Harnröhrenepithel keine schmerzhafte Reizung oder Trauma verursacht. In der Regel ist jedoch eine vollständig elastische Retrahierung des langfristig zum Ballon aufgedehnten Ballonelementes auf den Schaft nicht gegeben. Der partielle Verlust der Manschetten-Elasizität durch eine Aufdehnung des Ballonelementes über mehrere Tage kann durch chemisch aggressive Urinbestandteile (z.B. Harnsäure) beschleunigt werden. Bei Kathetern auf Latex-Basis führt der Urin bei längerfristiger Anwendung regelmäßig nicht nur zu einer ausgeprägten Versteifung des Ballonelementes, sondern auch zu einem erheblichen Verlust der Elastizität des Katheterschaftes. Ballonelemente bekannter Bauart auf der Basis einer Latex- oder Silikon-Manschette weisen nach der Entleerung in vielen Fällen residuale, grobe Falten bzw. Wülste in der sich (nicht vollständig) retrahierenden Hülle auf und stellen für den Patienten ein nicht unerhebliches Verletzungspotential dar.

Die bisher gebräuchlichen Katheter-Materialen (Latex, Silikon bzw. Latex- oder Silikon-basierte Materialien, bzw. Verbundmaterialien aus Latex und Silikon) weisen darüber hinaus zusätzliche klinisch relevante Nachteile auf.

Ein Nachteil (insbesondere beim Einsatz von Latexmaterialien) besteht darin, dass das Ballonelement sich beim Aufdehnen nicht immer formsymmetrisch entwickelt und leicht seitlich gewichtet ausbricht. Die Stabilität der Verankerung des Ballons im Blasenausgang kann durch eine ausgeprägte Formasymmetrie des Ballons beeinträchtigt sein. Eine ausgeprägte Asymmetrie des befüllten Ballonelementes kann bei entsprechender Platzierung im Blasenausgang darüber hinaus zum Abknicken des Katheterlumens führen.

Von Nachteil ist schließlich auch, dass das Ballonelement von Kathetern konventioneller Bauart, durch die Besonderheiten der Fertigung und des Materials bedingt, bestimmte Wandstärken nicht unterschreiten darf. Die sich beim Befüllen zum Ballon aufdehnende Manschette muss eine solche Mindest-Wandstärke aufweisen, um bei zunehmender Ausformung des Ballons (und der mit der Aufdehnung einhergehenden Reduktion der Ballon-Wandungsstärke) das Unterschreiten einer unteren kritischen, rupturgefährenden Mindest-Wandungsstärke sicher zu vermeiden. Die Mindest-Wandstärke des dem Schaft manschettenartig anliegenden Ballonelementes liegt üblicherweise im Bereich von mindestens 100 Mikrometer und erfordert bei der Aufdehnung bzw. Deformation der Manschette zum Ballon relativ hohe Drucke. Das Ballonelement formt sich bei seiner Entwicklung überwiegend in radialer, aber auch in longitudinaler Richtung aus (Streckung). Durch die bei vorwiegend radialer Ausdehnung der Ballonhülle entstehenden Drucke kommt es, mit steigendem Füllvolumen, in vielen Fällen zu einer Komprimierung bzw. Stenosierung des Drainagelumens des Katheters. Dieser lumeneinengende Effekt wird durch die ebenfalls stattfindende Ausdehnung des Ballons in longitudinaler Richtung bzw. die damit einhergehende Längsstreckung des Katheterschaftes im Ballonbereich unterstützt. Beide Dehnungskomponenten können zu einer erheblichen Taillierung bzw. Stenosierung des Katheterlumens führen.

Die Herstellung konventioneller Blasenkatheter ist aufwendig und erfordert viele einzelne Fertigungsschritte. Die jeweiligen Tauch- bzw. Gussverfahren gewährleisten in vielen Fällen keine ausreichende Oberflächengüte von Katheter und Ballon. Vor allem bei der Verarbeitung von Silikon ergeben sich leicht raue und unebene Grenzflächen, was die Anlagerung (Inkrustation) von Harnbestandteilen sowie die bakterielle Besiedlung von Katheteroberflächen begünstigt.

Bei der Verwendung von Silikon stellt sich zudem die besondere Problematik einer nicht unerheblichen Permeation von Wasser durch die Ballonhülle. Zur Sicherung einer suffizienten Ballonfüllung muss der Ballon in der Regel im beinahe täglichen Rhythmus nachbefüllt werden.

Die Dokumente EP 0 935 977 A2 und US-A-5,417,657 beschreiben jeweils einen Blasenkatheter mit einer aufblasbaren Manschette am vorderen Ende, die aus Polyurethan bestehen kann. In beiden Fällen ist die ballonartige Manschette derart elastisch ausgebildet, dass sich der betreffende Ballon vollständig elastisch zusammenziehen kann.

Es ist daher erforderlich, die Ballone entgegen der beträchtlichen, rückstellenden Kräfte der Ballonwandungen mit erheblichem Druck aufzudehnen. Dabei wirkt dieser große Druck auch auf den Katheterschaft ein, der bei Blasenkathetern elastisch, d.h. weich ist, damit er über längere Zeiträume hinweg in einem Patienten verbleiben kann, und preßt das zentrale Lumen darin zusammen. Die natürliche Drainage durch den Katheter wird dadurch beeinträchtigt oder gar unterbunden.

Außerdem bedingt der große Druck eine große Wandstärke des Ballons, um diesen Druck ohne Beschädigung ertragen zu können. Der Ballon kann daher nicht auf einen kleinen Durchmesser zusammengezogen werden, und das Einführen wie auch das Entfernen eines solchen Katheters kann daher zu Verwundungen der Harnröhre führen und ist sehr schmerzhaft.

### Darstellung der Erfindung

Aus den Nachteilen des beschriebenen Standes der Technik resultiert das die Erfindung initiierende Problem, einen gattungsgemäßen Blasenkatheter derart weiterzubilden, dass dieser über einen längeren Zeitraum hinweg tragbar ist, gute Drainageeigenschaften hat und möglichst schmerzfrei eingesetzt und wieder entfernt werden kann.

Die Lösung des gestellten Problems gelingt bei einem gattungsgemäßen Blasenkatheter dadurch, dass das Ballonelement eine blasgeformte Präformierung in Form eines Basisballons aufweist, dessen Ballonhülle nicht unter Spannung steht, und der, frei entfaltet, im ungedehnten Zustand eine Wandstärke von 5 bis 20 Mikrometer hat und bis zum Vollballon elastisch dehnbar ist.

Zwar ist die Fertigung eines Polyurethanschaftes durch das Verfahren der Extrusion bei Blasenkathetern bereits bekannt und im klinischen Einsatz am Patienten bewährt. Für das Ballonelement konventioneller Bauart galt Polyurethan wegen seines unzureichenden Dehnungsverhaltens allerdings als nicht geeignet.

Katheter mit Polyurethanschaft wurden bislang mit Ballonelementen aus Latex oder Silikon bzw. verwandten, ähnlich volumendehnbaren Materialien versehen. Die Aufdehnung einer über den Schaft gezogenen, dem Schaft (Schaftdurchmesser von üblicherweise ca. 4 bis 6 mm beim Erwachsenen) eng anliegenden Polyurethan-Manschette zum Ballon ausreichender Größe (Füllvolumen 5 bzw. 30 ml) könnte nur unter sehr hohem Druck erfolgen, der vom Anwender mit herkömmlichen Mitteln nur bedingt zu erzeugen wäre. Die in der Wandung des sich ausformenden Ballons entstehende Spannung wäre in jedem Falle beträchtlich. Das Drainagevolumen des Katheters wäre durch den immensen Ballonfülldruck, wie zuvor beschrieben, weitgehend eingeengt.

Überraschenderweise zeigte sich jedoch, dass die Verwendung eines Polyurethan-Ballonelementes bei der Fertigung eines Blasenkatheters dennoch möglich ist, insbesondere dann, wenn das Ballonelement zum Ballon vorgeformt, als Ballonfolie mit einer Wandstärke von 5 bis 20 Mikrometer, vorzugsweise 5 bis 15 Mikrometer, ausgebildet ist. Der erfindungsgemäße, vorzugsweise partiell vorgeformte Basis-Ballon liegt der Schaft-Wandung im entleerten Zustand unter Faltung seiner Hülle eng an. Der vorgeformte Basis-Ballon ist in bekannter Weise mit zwei Schaftstücken versehen, über die er am Katheterschaft befestigt wird. Eine Reduktion des Schaftdurchmessers, welche den auf dem Schaft in Faltung aufsitzenden bzw. auftragenden Basis-Ballon berücksichtigt, ist bei der Erfindung nicht notwendig. Der Anwender kann die Schaftstärke des Katheters in gewohnter Weise und ohne Einschränkungen wählen.

Um den heute gebräuchlichen Kathetertypen zu entsprechen, schlägt die Erfindung zwei grundlegende, partiell vorgeformte, sog. Basis-Ballon-Typen vor, die im vollständig gefüllten, vollständig ausgeformten Zustand (Arbeits-Ballon) ein Füllvolumen von 5 ml beziehungsweise 30 ml aufweisen..

Um ein Arbeits-Füllvolumen von 5 ml mit komfortablen, den Katheterschaft nicht komprimierenden Fülldruckwerten erreichen zu können, wird der Basisballon derart gestaltet, dass er im ungedehnten Ruhe- bzw. Basiszustand, d.h. bei Füllung des Ballons bis zur frei entfalteten Ruhe- bzw. Basisform (vorzugsweise Kugel- oder Spindelform) ein Ruhe- bzw. Basisvolumen von ca. 1,2 bis 2,5 ml aufweist. Die Cuff-Hülle ist in diesem gefüllten Basis-Zustand noch ungedehnt.

Der Ballon wird bevorzugt in längsgestreckter Form auf dem Schaft aufgebracht. Die Schaftstücke des Ballons werden dabei unter Vermeidung einer Zug-Dehnung der Ballonhülle, maximal voneinander beabstandet, auf dem Schaft fixiert. Die Ballonhülle richtet sich in einer schaft-parallelen Längsfaltung aus und liegt dem Katheterschaft eng angeschmiegt an. Das im derart aufgebrachten Ballon verbleibende Rest-Füllvolumen liegt in der Regel unter 0,05 ml, bevorzugt im Bereich von nur 0,01 bis 0,03 ml.

Die Wandstärke der Ballonhülle liegt bei der 5 ml-Arbeits-Füllvolumen-Ausführungsform bevorzugt im Bereich von 5 bis 10 Mikrometern.

Bei einem größeren Arbeits-Füllvolumen von beispielsweise 30 ml erhält der Basisballon im ungedehnten Basis-Zustand, d.h. bei Füllung des Ballons zur frei entfalteten Ruheform (vorzugsweise Walzen- oder Kugelform), ein Ruhevolumen von ca. 4 bis 10 ml, Der Ballon wird (in entsprechender Weise zum 5 ml-Ballon) bevorzugt in längsgestreckter Form auf dem Schaft aufgebracht. Das Restvolumen des derart aufgebrachten Cuffs liegt in der Regel unter 0,08 ml, bevorzugt im Bereich von nur 0,02 bis 0,04 ml. Die Wandstärke der Ballonhülle liegt bei der 30 ml-Arbeits-Füllvolumen-Ausführungsform bevorzugt im Bereich von 5 bis 15 micron.

Das eingesetzte Polyurethan-Polymer, das Ruhevolumen des Basisballons und die Wandstärke des Ballons werden so gewählt, dass der Sicherheitsbereich der Volumendehnbarkeit des Ballons bevorzugt 300 bis 400 Prozent beträgt und einen Sicherheitsbereich von 400 bis 450 Prozent nicht überschreitet.

Die in längs- oder auch in unausgerichteter Faltung geschlagene Ballonhülle gestattet die Ausdehnung eines partiell vorgeformten Basisballons zum befüllten Arbeits-Ballon mit komfortablen, das Katheterlumen nicht einengenden Druckwerten. Der Fülldruck im Ballon beträgt beim erfindungsgemäß vorgeformten Ballon in der Regel nur 50-200 mbar (bei 5 ml Arbeitsvolumen) bzw. 50-250 mbar (bei 30 ml Arbeitsvolumen.

Für den erfindungsgemäßen Ballon werden vorzugsweise die Materialien Pellethane 2363 mit einer Shore-Härte von 70 bis 90 mit ihren jeweiligen Unterformen (A, AE) verwendet. Materialien anderer Hersteller mit vergleichbaren technischen Materialdaten können entsprechend verwendet werden.

Der Ballon wird mit seinen Schaftstücken auf dem Katheterschaft aufgeklebt oder aufgeschweißt. Bei der Herstellung des Basis-Ballons werden die Übergangsbereiche von den Schaftstücken zum zentralen mittelständigen Durchmesser des Basisballons in ihren Wandstärken so ausgebildet, dass die Wandstärken kontinuierlich vom Schaftstück zum zentralen, mittelständigen Durchmesser hin abnehmen.

Es ist günstig, wenn nach der Verbindung des Ballons auf dem Katheterschaft die Endränder der Schaftstücke z.B. durch Hitzeeinwirkung oder Lösungsmittelauftrag geglättet werden, so dass hier keine scharfkantigen Stufen im Bereich des Übergangs von Schaft zum Ballon vorhanden sind.

Die Verwendung von Polyurethan für den Katheterschaft erschließt ferner den Vorteil, dass die Wandstärke des Katheterschaftes geringer als bisher ausgeführt wird, das Katheterdrainagelumen also bei gleichem Außendurchmesser vergrößert werden kann. So reicht bei günstigster Materialwahl eine Schaftwandstärke von 0,4 bis 0,8 mm, vorzugsweise von 0,4 bis 0,6 mm aus. Der Katheterschaft behält trotzdem seine zur Einführung in die Harnröhre erforderliche Steifigkeit bzw. Knicksicherheit bei der Anwendung im Patienten. Zur weiteren Reduktion der Katheterwandstärke wird der Katheterschaft bevorzugt aus zwei konzentrisch extrudierten Röhren gebildet, wobei die innere Röhre vorzugsweise dünner und härter als die äußere Röhre ausgestaltet ist (Co-Extrusion). Mit der gleichen Zielsetzung ist eine Spiralarmierung oder ein in den Schaft eingearbeitetes stabilisierendes Netz denkbar.

Darüber hinaus ist die Oberfläche sowohl des vorzugsweise im blow-molding-Verfahren hergestellten Ballons bzw. des bevorzugt extrudierten Schaftes bei Verwendung von Polyurethan von höchster Güte. Wegen der besonderen Ebenheit der Oberfläche wird die Anlagerung von Harnbestandteilen (Inkrustation) bzw. die baktierielle Kolonisation erschwert.

Die Fertigung des erfindungsgemäß beschriebenen Katheters ist produktionstechnisch einfach umzusetzen und erspart im Vergleich zu konventionellen Kathetertypen kostenintensive Fertigungsschritte, wie dies vor allem beim im Tauchverfahren hergestellten Latexkatheter der Fall ist.

Der Katheterschaft wird in dem vom Ballon erfassten Bereich bevorzugt mit mehreren Füllöffnungen versehen. Diese Füllöffnungen werden in einer eckigen, vorzugsweise rechteckigen Form ausgebildet. Hierdurch wird weitgehend verhindert, dass die dünne Folie des Ballons diese Öffnung bzw. diese Öffnungen ventilartig verschließen kann und das Entleeren des Ballons so erschwert.

Die Dimensionierung des Basisballons bzw. seiner Wandstärke wird so gewählt, dass die Hülle unter Vermeidung einer nicht-elastischen Überdehnung bis zum Arbeitsvolumen aufgedehnt werden kann bzw. die Elastizität des Ballonmaterials auch bei langfristigem Gebrauch des Katheters vollständig erhalten bleibt. Nach völliger Entleerung des Ballons liegt er daher dem Katheterschaft beim Zurückziehen durch die Harnröhre wiederum in längsgerichteter Faltung eng angeschmiegt an und verhält sich atraumatisch.

Der sogenannte suprabubische Blasenkatheter, eine weitere in der Praxis gängige Ausführungs- und Anwendungsform eines Blasenkatheters, kann ebenfalls durch die Kombination eines Polyurethanschaftes mit einem erfindungsgemäßen polyurethanbasierten Ballonelement optimiert werden. Die suprabubische Katheteranlage erfolgt durch den Einstich eines Hohl-Nadelelementes durch die äußere Bauchdecke hindurch direkt in die Harnblase, unmittelbar oberhalb des Schambeins. Das Nadelelement kann als konventionelle Hohlnadel, als seitlich durchgehend über die gesamte Nadellänge geöffnete Führungsnadel (der Katheter wird hier seitlich in die Führungsnadel eingelegt) oder z.B. als sogenannte Spreiznadel (die Hohlnadel besteht aus zwei durch Spreizung von einander separierbaren Hälften) ausgebildet sein.

Präformierte Ballonelemente, in der erfindungsgemäßen Ausführungsform des Ballonelementes mit einem Arbeits-Volumen von 5 ml im Wandstärkenbereich von vorzugsweise 5 bis 10 Mikrometern können bei Anwendung eines Gleitmittels ohne Einschränkung des Katheterschaftdurchmessers durch die Applikationsnadel geschoben werden. Die zuvor beschriebenen Vorteile des vollständig aus Polyurethan gefertigten Blasenkatheters können so auch bei der suprabubischen Applikationsform für den Patienten erschlossen werden.

Für den in der Regel über lange Perioden suprabubisch katheterisierten Patienten ist zudem entscheidend, dass das entleerte Ballonelement durch den nach längerer Anlage des Katheters bereits verheilten bzw. granulierten Stichkanal in Blase und Bauchwand möglichst atraumatisch zurückgezogen werden kann.

### Kurzbeschreibung der Zeichnung

Anhand der in der Zeichnung dargestellten Ausführungsbeispiele wird die Erfindung nachstehend näher erläutert.

Es zeigt:
- Figur 1: eine Seitenansicht des oberen Endes des Katheters teilweise im Schnitt,
- Figur 2: eine Seitenansicht des Katheters vor seinem Einsatz und
- Figur 3: eine Seitenansicht des Katheters vor dem Einführen beziehungsweise Herausziehen aus der Harnröhre.

### Ausführung der Erfindung

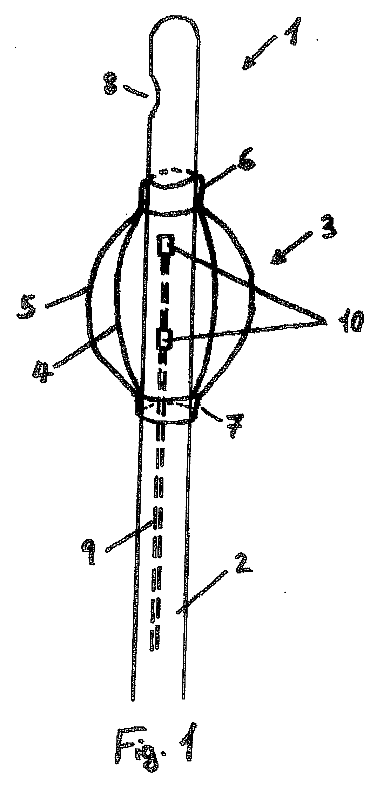

Die Figur 1 zeigt das vordere Ende eines Blasenkatheters 1 in der Seitenansicht, teilweise im Schnitt. Auf dem Katheterschaft 2 ist das Ballonelement 3 angebracht, das im Schnitt einmal als Basisballon 4 (Ruhevolumen) und einmal als Vollballon 5 (Arbeitsvolumen) gezeigt ist. Das Ballonelement 3 besteht aus einem Material auf Polyurethanbasis, es hat in seiner Form als Basisballon 4 eine Wandstärke von 5 bis 20, bevorzugt 5-15 µm. Es ist mit den Schaftstücken 6 und 7 versehen, über die es mit dem Katheterschaft 2 verklebt ist. Der hohle Katheterschaft 2 hat an seinem äußersten Ende die Öffnung 8, über die ham aus der Harnblase ausfließen kann ein in der Wandung des Katheterschaftes 2 befindlicher Auffüllkanal 9 führt zu der Öffnung oder mehreren Öffnungen 10 im Katheterschaft 2, die im Bereich des Ballonelementes 3 angebracht ist.

Durch eine geeignete Flüssigkeit, die durch den Kanal 9 und die Öffnung(en) 10 in das Ballonelement 3 geleitet wird, wird das Ballonelement 3 nach Einführung des Katheters 2 in die Harnblase durch die Harnröhre mit Flüssigkeit gefüllt, das heißt, unter zunehmender Füllung vom Basisvolumen auf sein Arbeitsvolumen aufgedehnt.

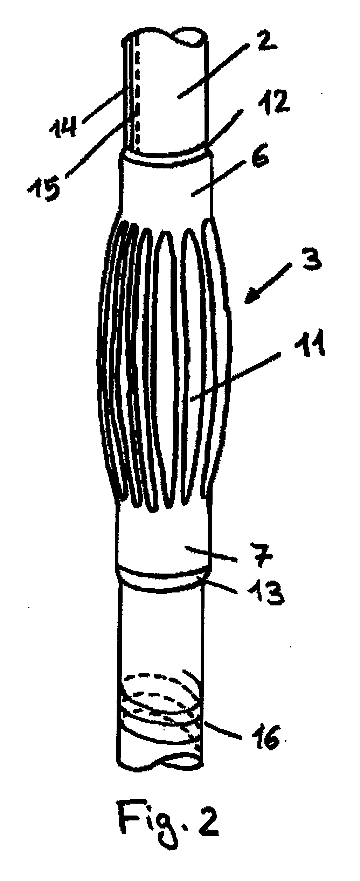

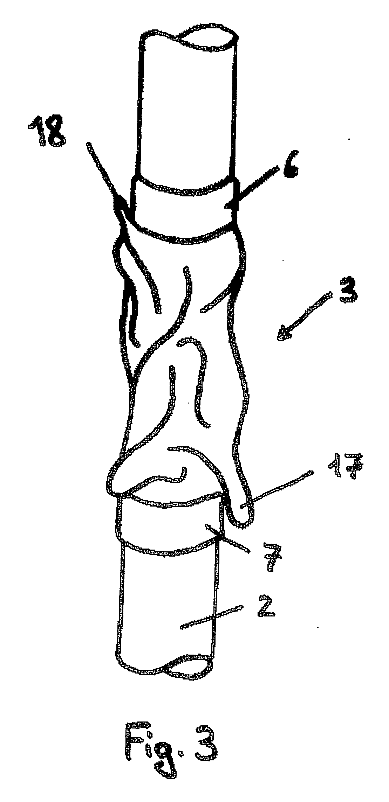

Vollständig entleert liegt das Ballonelement 3 an der Oberfläche des Schaftes 2 an, wie dies in der Figur 2 und Figur 3 gezeigt ist.

In Figur 2 bildet das Ballonelement 3 eine in Längsrichtung des Katheterschaftes 2 verlaufende Faltung 11. Die Faltung 11 erstreckt sich m wesentlichen zwischen den beiden Schaftstücken 6 und 7.

Die Faltenbildung läßt eine Auswölbung des Ballonelementes 3 zu, die zu dem in Figur 1 gezeigten Ballon 4 führt Diese Auswölbung geschieht ohne nennenswerten Druck und kann in Abhängigkeit vom eingesetzten Material unterschiedlich groß sein. Der Basisballon 4 enthält im ungedehnten Zustand frei entfaltet ein Ruhevolumen, das deutlich unter dem Füllvolumen liegt, welches der gefüllte Ballon 5 (Arbeitsvolumen) aufweist. Zur Veranschaulichung der Erfindung ist in der Figur 1 der Basisballon 4 mit einem relativ großen Ruhevolumen eingezeichnet. Um den Raumbedarf der Faltung so weit wie möglich zu reduzieren, wird der Basisballon in längsausgerichteter Form auf dem Schaft aufgebracht. Die Schaftstücke des Ballons werden, wie in Figur 2 gezeigt, so weit als möglich voneinander beabstandet, ohne dabei die Ballonhülle unter Spannung zu nehmen.

Der Übergangsbereich von den Schaftstücken 6 und 7 zum zentralen, mittelständigen Teil des Ballons 3 wird fließend gehalten, so dass die Wandstärken kontinuierlich von der Stärke an den Schaftstücken 6 und 7 zu der Stärke am zentralen, mittelständigen Durchmesser des Basisballons 4 abnehmen. Die Endränder 12 und 13 der Schaftstücke 6 und 7 sind geglättet, so dass kein scharfkantiger Übergang vorhanden ist.

Wie im oberen Teil der Figur 2 angedeutet, kann der Katheterschaft 2 aus zwei ineinanderliegenden, vorzugsweise co-extrudierten Röhren 14 und 15 bestehen.

Auf der unteren Hälfte der Figur 2 ist die Möglichkeit gezeigt, den Katheterschaft 2 mit einer Spiralarmierung 16 aus Metall zu versehen.

Die Figur 3 zeigt eine Ausführungsform des Ballonelementes 3, bei der der Basisballon 4 in nicht faltungsausgerichteter Form, sondern willkürlich gefaltet auf dem Schaft aufgebracht wird. Die Schaftstücke des Ballons sind hier also nicht maximal voneinander beabstandet, sondern in einem geringeren Abstand als dieser.

Die Faltenbildung kann so beliebig verlaufen, beispielsweise auch quer oder im rechten Winkel zur Katheterachse liegen. Da die Ballonwand jedoch außergewöhnlich dünn ist, kann sie sich, wenn der Ballon entleert ist, sehr eng an die Oberfläche des Katheterschaftes 2 anschmiegen. Gegebenenfalls entstehen Überwürfe 17 beziehungsweise 18 an den Schaftstücken 6 oder 7, wenn der Katheter ein- beziehungsweise ausgeführt wird. Auf der rechten Seite der Figur 3 ist mit 17 ein Überwurf angedeutet, der entsteht, wenn der Katheter eingeführt wird, und auf der linken Seite der Figur ist mit 18 ein Überwurf gezeigt, der entsteht, wenn der Katheter durch die Harnröhre zurückgezogen wird. Im erfindungsgemäß benannten Wandstärkenbereich wirken sich jedoch auch derartige Überwürfe bei der Passage des Ballonelementes durch die Harnröhre nicht nachteilig aus.

## Patentansprüche

1. Blasenkatheter (1) zur transurethralen oder suprabubischen Einführung in die Harnblase, bestehend aus einem elastischen Katheterschaft (2) mit einem daran befestigten, auffüllbaren Ballonelement (3) aus Polyurethan oder einer Polyurethan-Polyvinylchlorid-Mischung oder vergleichbarem Material auf Polyurethanbasis, das über wenigstens eine Füllöffnung (10) mit einem in die Wand des Katheterschaftes (2) eingebundenen Auffüllkanal (9) kommuniziert und durch Schaftstücke (6,7) mit dem Katheterschaft (2) verbunden ist, **dadurch gekennzeichnet, dass** das Ballonelement (3) eine blasgeformte Präformierung in Form eines Basisballons (4) aufweist, dessen Ballonhülle nicht unter Spannung steht, und der, frei entfaltet, im ungedehnten Zustand eine Wandstärke von 5 bis 20 Mikrometer hat und bis zum Vollballon (5) elastisch dehnbar ist.

2. Blasenkatheter nach Anspruch 1, **dadurch gekennzeichnet, dass** der Katheterschaft (2) aus Polyurethan oder einer Polyurethan-Polyvinylchlorid-Mischung oder einem vergleichbarem Material auf Polyurethanbasis besteht.

3. Blasenkatheter nach Anspruch 1 oder 2, **dadurch gekennzeichnet, dass** der Basisballon (4) eine Wandstärke von 5 bis 15 Mikrometer hat.

4. Blasenkatheter nach einem der Ansprüche 1 bis 3, **dadurch gekennzeichnet, dass** das Ballonelement (3) in seinem entleerten Ruhezustand an der Oberfläche des Katheterschaftes (2) unter Ausbildung einer Faltung anliegt.

5. Blasenkatheter nach einem der Ansprüche 1 bis 4, **dadurch gekennzeichnet, dass** das gefüllte Ballonelement (5) ein Gesamtfüllvolumen von 5 ml hat.

6. Blasenkatheter nach Anspruch 5, **dadurch gekennzeichnet, dass** der Basisballon (4) im ungedehnten, frei zum Ballon entfalteten Zustand ein Ruhevolumen von 1,2 bis 2,5 ml hat.

7. Blasenkatheter nach einem der Ansprüche 5 oder 6, **dadurch gekennzeichnet, dass** der Basisballon (4) in dem Katheterschaft (2) anliegenden, entleerten, längsgefalteten Zustand ein Restvolumen von weniger als 0,05 ml und bevorzugt von 0,01 bis 0,03 ml hat.

8. Blasenkatheter nach einem der Ansprüche 5 bis 7, **dadurch gekennzeichnet, dass** das Ballonelement (3) eine kugelige oder spindelförmige Gestalt hat.

9. Blasenkatheter nach einem der Ansprüche 1 bis 4, **dadurch gekennzeichnet, dass** das gefüllte Ballonelement (5) ein Gesamtfüllvolumen von 30 ml hat.

10. Blasenkatheter nach Anspruch 9, **dadurch gekennzeichnet, dass** der Basisballon (4) im ungedehnten, frei zum Ballon entfalteten Zustand ein Ruhevolumen von 4 bis 12 ml hat.

11. Blasenkatheter nach einem der Ansprüche 9 oder 10, **dadurch gekennzeichnet, dass** der Basisballon (4) in dem Katheterschaft (2) anliegendem, entleerten, längsgefalteten Zustand ein Restvolumen von weniger als 0,08 ml und bevorzugt 0,02 bis 0,04 ml hat.

12. Blasenkatheter nach einem der Ansprüche 9 bis 11, **dadurch gekennzeichnet, dass** der Ballon (3) eine sphärische oder walzenförmige Gestalt hat.

13. Blasenkatheter nach einem der Ansprüche 1 bis 12, **dadurch gekennzeichnet, dass** der Ballon (3) im Ruhezustand mit seinen Schaftstücken 86,7) auf den Katheterschaft aufgeklebt oder angeschweißt ist.

14. Blasenkatheter nach einem der Ansprüche 1 bis 13, **dadurch gekennzeichnet, dass** die Faltung des Ballons (3) parallel zum Schaft verlaufend ausgerichtet ist und ohne Dehnung des so gefalteten Ballons mit dem Katheterschaft (2) verbunden ist.

15. Blasenkatheter nach einem der Ansprüche 1 bis 14, **dadurch gekennzeichnet, dass** die Innendurchmesser der Schaftstücke (6,7) geringfügig kleiner sind als der Außendurchmesser der Katheterschafts (2).

16. Blasenkatheter nach einem der Ansprüche 1 , bis 15, **dadurch gekennzeichnet, dass** in den Übergangsbereichen von den Schaftstücken (6,7) zum Ballon (3) die Wandstärken kontinuierlich von der Stärke an den Schaftstücken (6,7) zu der Stärke im zentralen Abschnitt des Basisballons (4) abnehmen.

17. Blasenkatheter nach einem der Ansprüche 1 bis 16, **dadurch gekennzeichnet, dass** die Endränder (12,13) der Schaftstücke (6,7) geglättet sind.

18. Blasenkatheter nach einem der Ansprüche 1 bis 17, **dadurch gekennzeichnet, dass** der Katheterschaft (2) aus zwei ineinanderliegenden Röhren (14,15) besteht.

19. Blasenkatheter nach Anspruch 18, **dadurch gekennzeichnet, dass** die innere Röhre (15) dünner als die äußere Röhre (14) ausgebildet ist.

20. Blasenkatheter nach einem der Ansprüche 18 oder 19, **dadurch gekennzeichnet, dass** die innere Röhre (15) aus einem härteren Material als äußere Röhre (14) besteht.

21. Blasenkatheter nach einem der Ansprüche 1 bis 20, **dadurch gekennzeichnet, dass** wenigstens eine Füllöffnung (10) eine eckige oder vorzugsweise rechteckige Form aufweist.

22. Blasenkatheter nach einem der Ansprüche 1 bis 21, **dadurch gekennzeichnet, dass** der Katheterschaft (2) eine Spiralarmierung (16) aus Metall hat.

23. Blasenkatheter nach einem der Ansprüche 1 bis 22, **dadurch gekennzeichnet, dass** der Katheterschaft (2) durch Extrudieren hergestellt ist.

24. Blasenkatheter nach einem der Ansprüche 1 bis 23, **dadurch gekennzeichnet, dass** der Sicherheitsbereich der Volumendehnbarkeit des Ballons (3) maximal 400 bis 450 Prozent beträgt, vorzugsweise ca. 300 bis 400 Prozent.

25. Blasenkatheter nach einem der Ansprüche 1 bis 24, **dadurch gekennzeichnet, dass** die Ballonwand durch den Füllvorgang überdehnt wird und danach eine Wandstärke hat, die 5 bis 20 Mikrometer beträgt.

26. Blasen katheter nach einem der Ansprüche 1 bis 25, **dadurch gekennzeichnet, dass** der Fülldruck im 5ml-Ballon (5) 50 bis 200 Millibar beträgt und im 30ml-Ballon (5) 50 bis 250 Millibar.

## Claims

1. Bladder catheter (1) for transurethral or suprapubic introduction into the urinary bladder, comprised of a flexible catheter shaft (2) having a refillable balloon element (3) secured thereto and made of a polyurethane, a polyurethane-polyvinyl chloride blend, or of a comparable, polyurethane-based material, which communicates through at least one filling opening (10) with a filling channel (9) integrated in the wall of the catheter shaft (2), whereby the balloon element is fastened to the catheter shaft by shaft pieces (6,7), **characterised in that** the balloon element (3) is provided with a preformation by a blow-molding method in the form of a base-state balloon (4), the balloon envelope of which is not under tension, and which in the unexpanded, freely unfolded state, has a wall thickness of 5 to 20 micrometers and is elastically expandable up to the inflated balloon (5).

2. Bladder catheter according to claim 1, **characterised in that** the catheter shaft (2) is made of a polyurethane, a polyurethane-polyvinyl chloride blend, or of a comparable, polyurethane-based material.

3. Bladder catheter according to claim 1 or 2, **characterised in that** the base-state balloon (4) has a wall thickness of 5 to 15 micrometers.

4. Bladder catheter according to one of claims 1 through 3, **characterised in that** in its emptied, at-rest state, the balloon element (3) fits on the surface of the catheter shaft (2), forming folds.

5. Bladder catheter according to one of claims 1 through 4, **characterised in that** the fluid -inflated balloon element (5) has a total filling volume of 5 ml.

6. Bladder catheter according to claim 5, **characterised in that** in the unexpanded state, in which it is freely unfolded to form a balloon, the base-state balloon (4) has a volume at rest of 1.2 to 2.5 ml.

7. Bladder catheter according to claim 5 or 6, **characterised in that** in the drained, longitudinally folded state, in which it fits on the shaft, the base state balloon (4) has a volume at rest of less than 0.05 ml and preferably of 0.01 to 0.03 ml.

8. Bladder catheter according to one of claims 5 to 7, **characterised in that** the balloon element (3) has a spherical or spindle form.

9. Bladder catheter according to one of claims 1 to 4, **characterised in that** the fluid-inflated balloon element (5) has a total filling volume of 30 ml.

10. Bladder catheter according to claim 9, **characterised in that** in the unexpanded state, in which it is freely unfolded to form a balloon, the base-state balloon (4) has a volume at rest of 4 to 12 ml.

11. Bladder catheter according to one of claims 9 or 10, **characterised in that** in the drained, longitudinally folded state, in which it fits on the shaft, the base-state balloon (4) has a volume at rest of less than 0.08 ml and preferably of 0.02 to 0.04 ml.

12. Bladder catheter according to one of claims 9 to 11, **characterised in that** the filled balloon (5) has a spherical or cylindrical form.

13. Bladder catheter according to one of claims 1 to 12, **characterised in that** the balloon (3), in its at-rest state, is bonded or fused, via its shaft pieces (6, 7), to the catheter shaft.

14. Bladder catheter according to one of claims 1 to 13, **characterised in that** the fold formation of the balloon (3) is aligned in parallel with the shaft and is joined to the catheter shaft (2) without stretching the balloon folded in this manner.

15. Bladder catheter according to one of claims 1 to 14, **characterised in that** the inside diameter of the shaft pieces (6, 7) is slightly smaller than the outside diameter of the catheter shaft (2).

16. Bladder catheter according to one of claims 1 to 15, **characterised in that** in the transition regions from the shaft pieces (6, 7) to the balloon (3), the wall thickness continuously decreases from the thickness at the shaft pieces (6, 7) to the thickness in the center section of the base-state balloon (4).

17. Bladder catheter according to one of claims 1 to 16, **characterised in that** the end rims (12, 13) of the shaft pieces (6, 7) are smoothed.

18. Bladder catheter according to one of claims 1 to 17, **characterised in that** the catheter shaft (2) is composed of two tubes (14, 15), which fit one inside the other.

19. Bladder catheter according to claim 18, **characterised in that** the inner tube (15) is thinner than the outer tube (14).

20. Bladder catheter according to one of claims 18 or 19, **characterised in that** the inner tube (15) is made of a harder material than the outer tube (14).

21. Bladder catheter according to one of claims 1 to 20, **characterised in that** the at least one filling opening (10) has an angular or, preferably, rectangular shape.

22. Bladder catheter according to one of claims 1 to 21, **characterised in that** the catheter shaft (2) has a spiral reinforcement (16) of metal.

23. Bladder catheter according to one of claims 1 to 22, **characterised in that** the catheter shaft (2) is manufactured in an extrusion process.

24. Bladder catheter according to one of claims 1 to 23, **characterised in that** the safety range of volumetric expandability of the balloon (3) is 400 to 450 percent at a maximum, preferably approximately 300 to 400 percent.

25. Bladder catheter according to one of claims 1 to 24, **characterised in that** the balloon wall is stretched by the filling operation and, subsequently thereto, has a wall thickness of 5 to 20 µm.

26. Bladder catheter according to one of claims 1 to 25, **characterised in that** the filling pressure in the 5 ml balloon (5) is 50 to 200 millibar and, in the 0 ml balloon (5), 50 to 250 millibar.

## Revendications

1. Sonde vésicale (1) destinée à être introduite dans la vessie par voie transurétrale ou transabdominale, constituée d'une tige de sonde (2) élastique ayant, fixée à elle, un élément formant ballon (3), pouvant être rempli pour son gonflage, en polyuréthane ou en un mélange polyuréthane-poly(chlorure de vinyle) ou une matière comparable à base de polyuréthane, qui communique, au travers d'au moins un orifice de remplissage (10), avec un conduit de remplissage (9) intégré dans la paroi de la tige de sonde (2) et est relié à la tige de sonde (2) par des morceaux de tige (6, 7), **caractérisée en ce que** l'élément formant ballon (3) présente un préformage conformé à la vessie, sous forme d'un ballon de base (4), dont l'enveloppe ne se trouve pas sous tension et qui, librement déployé, a, dans l'état non étiré, une épaisseur de paroi de 5 à 20 micromètres et est expansible élastiquement jusqu'au ballon plein (5).

2. Sonde vésicale selon la revendication 1, **caractérisée en ce que** la tige de sonde (2) est réalisée en polyuréthane ou en un mélange polyuréthane-poly(chlorure de vinyle) ou une matière comparable à base de polyuréthane.

3. Sonde vésicale selon la revendication 1 ou 2, **caractérisée en ce que** le ballon de base (4) a une épaisseur de paroi de 5 à 15 micromètres.

4. Sonde vésicale selon l'une des revendications 1 à 3, **caractérisée en ce que** l'élément formant ballon (3), dans son état de repos vidé, vient s'appliquer sur la surface de la tige de sonde (2) en formant un plissement.

5. Sonde vésicale selon l'une des revendications 1 à 4, **caractérisée en ce que** l'élément formant ballon (5) rempli a un volume de remplissage total de 5 ml.

6. Sonde vésicale selon la revendication 5, **caractérisée en ce que** le ballon de base (4) a, dans l'état librement déployé jusqu'au ballon, non étiré, un volume de repos de 1,2 à 2,5 ml.

7. Sonde vésicale selon l'une des revendications 5 ou 6, **caractérisée en ce que** le ballon de base (4) a, dans l'état s'appliquant sur la tige de sonde (2), vidé et plissé longitudinalement, un volume résiduel de moins de 0,05 ml et, de préférence, de 0,01 à 0,03 ml.

8. Sonde vésicale selon l'une des revendications 5 à 7, **caractérisée en ce que** l'élément formant ballon (3) a une configuration sphérisée ou fuselée.

9. Sonde vésicale selon l'une des revendications 1 à 4, **caractérisée en ce que** l'élément formant ballon (5) rempli a un volume de remplissage total de 30 ml.

10. Sonde vésicale selon la revendication 9, **caractérisée en ce que** le ballon de base (4) a, dans l'état librement déployé jusqu'au ballon, non étiré, un volume de repos de 4 à 12 ml.

11. Sonde vésicale selon l'une des revendications 9 ou 10, **caractérisée en ce que** le ballon de base (4) a, dans l'état s'appliquant sur la tige de sonde (2), vidé et plissé longitudinalement, un volume résiduel de moins de 0,08 ml et, de préférence, de 0,02 à 0,04 ml.

12. Sonde vésicale selon l'une des revendications 9 à 11, **caractérisée en ce que** le ballon (3) a une configuration de forme sphérique ou cylindrique.

13. Sonde vésicale selon l'une des revendications 1 à 12, **caractérisée en ce que** le ballon (3), dans l'état de repos, est collé sur la tige de sonde ou soudé à celle-ci, par ses morceaux de tige (6, 7).

14. Sonde vésicale selon l'une des revendications 1 à 13, **caractérisée en ce que** le plissement du ballon (3) est orienté dans une direction parallèle à la tige et est relié à la tige de sonde (2) sans extension du ballon ainsi plissé.

15. Sonde vésicale selon l'une des revendications 1 à 14, **caractérisée en ce que** les diamètres intérieurs des morceaux de tige (6, 7) sont légèrement plus petits que le diamètre extérieur de la tige de sonde (2).

16. Sonde vésicale selon l'une des revendications 1 à 15, **caractérisée en ce que** dans les zones de transition des morceaux de tige (6, 7) au ballon (3), les épaisseurs de paroi diminuent, d'une façon continue, de l'épaisseur au niveau des morceaux de tige (6, 7) à l'épaisseur dans la partie centrale du ballon de base (4).

17. Sonde vésicale selon l'une des revendications 1 à 16, **caractérisée en ce que** les bords d'extrémité (12, 13) des morceaux de tige (6, 7) sont polis.

18. Sonde vésicale selon l'une des revendications 1 à 17, **caractérisée en ce que** la tige de sonde (2) est constituée de deux tubes (14, 15) placés l'un dans l'autre.

19. Sonde vésicale selon la revendication 18, **caractérisée en ce que** le tube intérieur (15) est réalisé plus mince que le tube extérieur (14).

20. Sonde vésicale selon l'une des revendications 18 ou 19, **caractérisée en ce que** le tube intérieur (15) est constitué d'une matière plus dure que le tube extérieur (14).

21. Sonde vésicale selon l'une des revendications 1 à 20, **caractérisée en ce qu'**au moins un orifice de remplissage (10) présente une forme angulaire ou, de préférence, rectangulaire.

22. Sonde vésicale selon l'une des revendications 1 à 21, **caractérisée en ce que** la tige de sonde (2) possède une armature hélicoïdale (16) en métal.

23. Sonde vésicale selon l'une des revendications 1 à 22, **caractérisée en ce que** la tige de sonde (2) est produite par extrusion.

24. Sonde vésicale selon l'une des revendications 1 à 23, **caractérisée en ce que** la plage de sécurité de l'expansibilité volumique du ballon (3) est au maximum de 400 à 450 pour cent, de préférence de 300 à 400 pour cent environ.

25. Sonde vésicale selon l'une des revendications 1 à 24, **caractérisée en ce que** la paroi du ballon est soumise à une sur-extension par l'opération de remplissage et a ensuite une épaisseur de paroi qui est de 5 à 20 micromètres.

26. Sonde vésicale selon l'une des revendications 1 à 25, **caractérisée en ce que** la pression de remplissage, dans le ballon de 5 ml (5), est de 50 à 200 millibars et, dans le ballon de 30 ml (5), de 50 à 250 millibars.
